# DEMANDE DE BREVET EUROPEEN

(11) **EP 1 655 299 A1**
(43) Date de publication de la demande: **10.05.2006**
(21) Numéro de dépôt: 05292309.1
(22) Date de dépôt: 02.11.2005
(51) Int. Cl.: C07D 417/12, A61K 31/5415

(54) **Dérivés de benzothiadiazine, leur procédé de préparation et les compositions pharmaceutiques qui les contiennent**

(30) Priorité: 03.11.2004 FR 0411691
(71) Demandeur: Les Laboratoires Servier, 92415 Courbevoie Cedex (FR)
(72) Inventeur: Desos, Patrice, 92270 Bois-Colombes (FR); Cordi, Alexis, 92150 Suresnes (FR); Lestage, Pierre, 78170 La Celle-St-Cloud (FR)

(57) **Abrégé**

Composés de formule (I) : 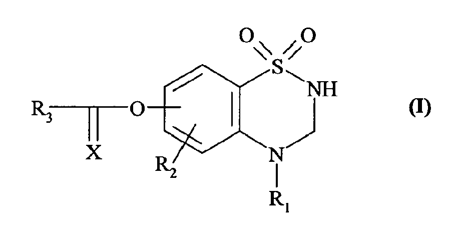 dans laquelle :
**X** représente un atome d'oxygène ou de soufre,
**R**_{**1**} représente un alkyle substitué par un ou plusieurs atomes d'halogène,
**R**_{**2**} représente hydrogène, halogène ou OH,
**R**_{**3**} représente **R**_{**4**} ou **Y- R**_{**5**} où **R**_{**4**}**, Y** et **R**_{**5**} sont tels que définis dans la description
Médicaments.

## Description

La présente invention concerne de nouveaux dérivés de benzothiadiazine, leur procédé de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit entre autres certains dérivés de benzothiadiazine en tant que modulateurs des récepteurs AMPA.

Les dérivés de benzothiadiazine, objets de la présente invention, outre le fait qu'ils soient nouveaux, présentent, de manière surprenante, des activités pharmacologiques sur le courant AMPA particulièrement intéressantes. Ils sont utiles en tant que modulateurs AMPA pour le traitement ou la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule **(I)** : 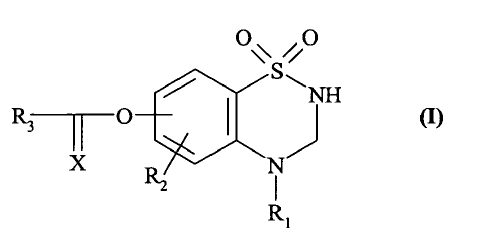 dans laquelle :
- **X**: représente un atome d'oxygène ou de soufre,
- **R**_{**1**}: représente un groupement alkyle (C₁-C₆) linéaire ou ramifié substitué par un ou plusieurs atomes d'halogène,
- **R**_{**2**}: représente un atome d'hydrogène ou d'halogène ou un groupement hydroxy,
- **R**_{**3**}: représente un groupement R₄ ou -Y-R₅ dans lesquels
R₄ représente un groupement alkyle (C₁-C₆) linéaire ou ramifié éventuellement substitué par un groupement aryle ; alkényle (C₂-C₆) linéaire ou ramifié éventuellement substitué par un groupement aryle ; polyhalogénoalkyle (C₁-C₆) linéaire ou ramifié ; cycloalkyle (C₃-C₇) ; adamantyle ; aryle ou hétéroaryle,
Y représente un atome d'oxygène ou de soufre ou un groupement NR dans lequel R représente un atome d'hydrogène ou un groupement alkyle (C₁-C₆) linéaire ou ramifié, et R₅ peut prendre toutes les valeurs de R₄,
leurs énantiomères et diastéréoisomères ainsi que leurs sels d'addition à un acide ou à une base pharmaceutiquement acceptable,
étant entendu que :
◆ par groupement aryle, on comprend groupement aromatique monocyclique ou un groupement bicyclique dans lequel au moins un des cycles est aromatique, éventuellement substitué par un ou plusieurs groupements, identiques ou différents, halogène, alkyle (C₁-C₆) linéaire ou ramifié, alkoxy (C₁-C₆) linéaire ou ramifié, polyhalogénoalkyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements, identiques ou différents, choisis parmi alkyle (C₁-C₆) linéaire ou ramifié et acyle (C₁-C₆) linéaire ou ramifié), alkyl (C₁-C₆) sulfonylamino, ou phényle (éventuellement substitué par un ou plusieurs groupements, identiques ou différents, halogène, alkyle (C₁-C₆) linéaire ou ramifié, polyhalogénoalkyle (C₁-C₆) linéaire ou ramifié, hydroxy ou alkoxy (C1-C6) linéaire ou ramifié),
◆ par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène et soufre, éventuellement substitué par un ou plusieurs groupements, identiques ou différents, halogène, alkyle (C₁-C₆) linéaire ou ramifié, alkoxy (C₁-C₆) linéaire ou ramifié, polyhalogénoalkyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements, identiques ou différents, choisis parmi alkyle (C₁-C₆) linéaire ou ramifié et acyle (C₁-C₆) linéaire ou ramifié), ou alkyl (C₁-C₆) sulfonylamino.

X représente plus préférentiellement un atome d'oxygène.
Le groupement R₁ préféré est l'halogénoéthyle comme par exemple le fluoroéthyle, le chloroéthyle ou le bromoéthyle.
R₂ représente avantageusement un atome d'hydrogène.
Le groupement R₃ préféré est le groupement aryle ou hétéroaryle.

Plus particulièrement, l'invention concerne les composés de formule (I) qui sont :
- le 3-thiophènecarboxylate de 4-(2-fluoroéthyl)-1,1-dioxido-3,4-dihydro-2*H*-1,2,4-benzothiadiazin-7-yle, et
- le benzoate de 4-(2-fluoroéthyl)-1,1-dioxido-3,4-dihydro-2*H*-1,2,4-benzothiadiazin-7-yle.

L'invention concerne également le procédé de synthèse de composés de formule (I) caractérisé en ce que l'on utilise comme produit de départ le composé de formule (II) : 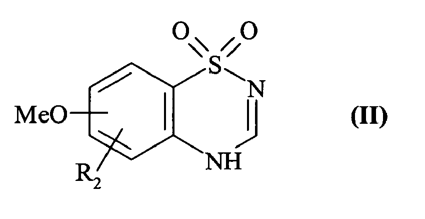 dans laquelle R₂ est tel que défini dans la formule (I),
sur lequel on condense, en milieu basique, un halogénoalkyle(C₁-C₆) linéaire ou ramifié comportant une fonction hydroxyle,
que l'on transforme ensuite en dérivé halogéné correspondant pour conduire au composé de formule (III) : 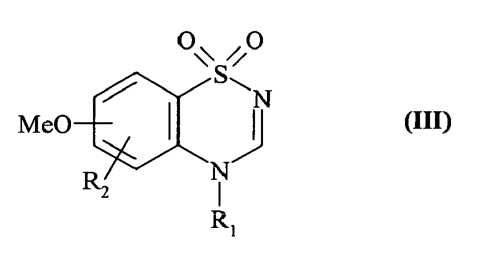 dans laquelle R₁ et R₂ sont tels que définis dans la formule (I),
que l'on soumet à une réduction, en présence de NaBH₄ par exemple, pour obtenir le composé de formule (IV) : 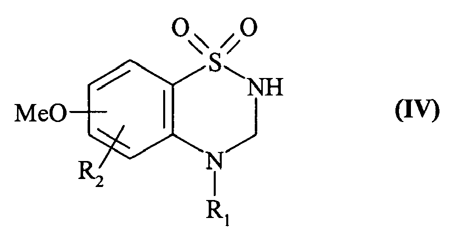 dans laquelle R₁ et R₂ sont tels que définis précédemment,
que l'on soumet à une réaction de déméthylation, en présence de BBr₃ ou BF₃ par exemple, pour conduire au composé de formule (V) : 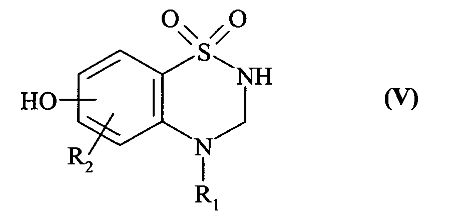 dans laquelle R₁ et R₂ sont tels que définis précédemment,
composé de formule (V) pouvant être également obtenu à partir du composé de formule (III) que l'on soumet à une réaction de déméthylation, en présence de BBr₃ ou BF₃ par exemple, pour conduire au composé de formule (VI) : 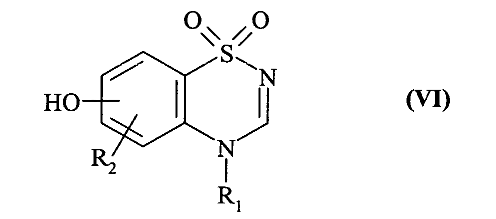 dans laquelle R₁ et R₂ sont tels que définis précédemment,
que l'on soumet à une réduction, en présence de NaBH₄ par exemple, pour obtenir le composé de formule (V) tel que défini précédemment,
sur lequel on condense le composé de formule (VII) : 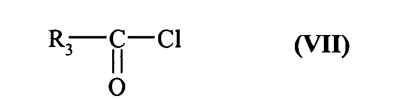 dans laquelle R₃ est tel que défini dans la formule (I)
pour obtenir le composé de formule (I/a), cas particulier des composés de formule (I) : 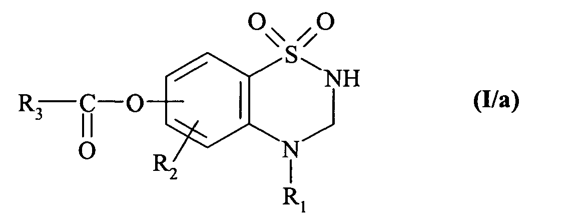 dans laquelle R₁, R₂ et R₃ sont tels que définis précédemment,
que l'on soumet à un agent de thionation, comme le réactif de Lawesson par exemple, pour conduire au composé de formule (I/b), cas particulier des composés de formule (I) : 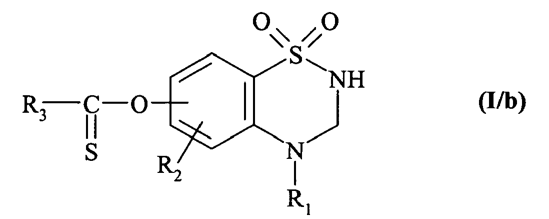 dans laquelle R₁, R₂ et R₃ sont tels que définis précédemment,
les composés de formule (I/a) et (I/b) formant l'ensemble des composés de formule (I) que l'on purifie, le cas échéant, selon une technique classique de purification, dont on sépare éventuellement les isomères, selon une technique classique de séparation, et que l'on transforme, si on le souhaite, en leurs sels d'addition, à un acide ou à une base pharmaceutiquement acceptable.

L'invention concerne également le composé de formule (V) : 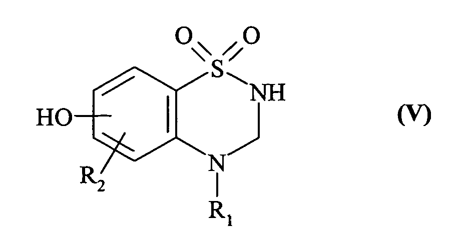 tel que défini précédemment, utile en tant qu'intermédiaire de synthèse pour la synthèse des composés de formules (I) et utile en tant qu'agent modulateur des récepteurs AMPA,
et plus particulièrement le composé de formule (VIII), cas particulier de composés de formule (V) : 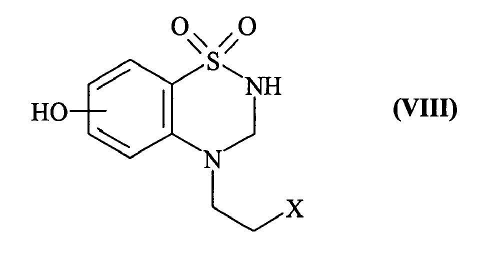 dans laquelle X représente un atome de fluor, de chlore, de brome ou d'iode utile en tant qu'intermédiaire de synthèse pour la synthèse des composés de formules (I) et utile en tant qu'agent modulateur des récepteurs AMPA.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les préparations et exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse...)

### Préparation 1 : 4-(2-Bromoéthyl)-3,4-dihydro-2H-1,2,4-benzothiadiazin-7-ol 1,1-dioxide

### Stade A : 2-(7-Méthoxy-1,1-dioxido-4H-1,2,4-benzothiadiazin-4-yl)éthanol

A une solution de 7-méthoxy-4*H*-1,2,4-benzothiadiazine 1,1-dioxide (4,0 g, 18,8 mmol) dans un mélange de 30 ml de DMF et 30 ml de CH₃CN, on ajoute 8,6 g (56,6 mmol) de CsF et 1,47 ml (18,8 mmol) de 2-bromoéthanol. On agite 2 h à 75 °C et on rajoute 1,47 ml (18,8 mmol) de 2-bromoéthanol. Après 6 h supplémentaires à 75 °C on rajoute à nouveau 1,47 ml (18,8 mmol) de 2-bromoéthanol puis 2,8 g (18,8 mmol) de CsF et poursuit l'agitation à 75 °C pendant une nuit. On filtre les sels à température ambiante, rince avec du CH₃CN, évapore le filtrat à sec . On reprend le résidu dans CH₂Cl₂, lave la phase organique avec du NaCl saturé, sèche (MgSO₄). Après évaporation, le résidu collant est repris dans un mélange éther éthylique / CH₂Cl₂. On triture la gomme jusqu'à obtention d'un solide que l'on filtre pour obtenir le produit du titre.
*Point de fusion : 160-162 °C*
*Microanalyse élémentaire :*

| | *C* | *H* | *N* | *S* |
|---|---|---|---|---|
| *% théorique* | *46,87* | *4,72* | *10,93* | *12,51* |
| *% expérimental* | *46,99* | *4,96* | *10,34* | *12,51* |

### Stade B : 4-(2-Fluoroéthyl)-7-méthoxy-4H-1,2,4-benzothiadiazine 1,1-dioxide

A une solution de 3,85 g (15,02 mmol) du produit du stade précédent dans 100 ml de CH₂Cl₂ refroidis dans un bain de glace, on rajoute goutte à goutte 3,97 ml (30,0 mmol) de DAST dilué dans 20 ml de CH₂Cl₂. On laisse ensuite la solution réactionnelle revenir à température ambiante en 1 h environ puis on verse 100 ml de NaCl saturée, décante la phase organique, sèche (MgSO₄), évapore sous vide. Le résidu est trituré dans un mélange éther éthylique/ CH₂Cl₂ jusqu'à obtention d'un solide que l'on filtre pour obtenir le produit du titre.
*Point de fusion : 123-128 °C*
*Microanalyse élémentaire :*

| | *C* | *H* | *N* | *S* |
|---|---|---|---|---|
| *% théorique* | *46,50* | *4, 29* | *10,85* | *12,42* |
| *% expérimental* | *45,88* | *4,41* | *10,46* | *12,61* |

### Stade C : 4-(2-Fluoroéthyl)-7-méthoxy-3,4-dihydro-2H-1,2,4-benzothiadiazine 1,1-dioxide

A une suspension de 2,77 g (10,7 mmol) du produit du stade précédent dans 25 ml d'éthanol, on rajoute par petites portions 454 mg (12,0 mmol) de NaBH₄. Après 2 h d'agitation à température ambiante on ajoute goutte à goutte de l'HCl 1N jusqu'à formation d'un précipité blanc que l'on filtre pour récupérer le produit du titre.
*Point de fusion : 91-93 °C*

### Stade D : 4-(2-Bromoéthyl)-3,4-dihydro-2H-1,2,4-benzothiadiazin-7-ol 1,1-dioxide

A une solution de 1,53 g (5,88 mmol) du produit du stade précédent dans 70 ml de CH₂Cl₂ refroidis dans un bain de glace, on ajoute goutte à goutte 17,6 ml (17,6 mmol) d'une solution 1M de BBr₃ dans CH₂Cl₂. On agite une nuit en laissant revenir à température ambiante. La suspension réactionnelle est refroidie dans un bain de glace et on ajoute goutte à goutte 50 ml d'eau. Après 30 min d'agitation on filtre le précipité, le rince à l'eau et le sèche sous vide . On obtient ainsi le produit attendu sous forme d'une poudre marron clair.
*Point de fusion : 144-148 °C*

### Préparation 2 : 4-(2-Fluoroéthyl)-3,4-dihydro-2H-1,2,4-benzothiadiazin-7-ol 1,1-dioxide

### Stade A : 4-(2-Fluoroéthyl)-4H-1,2,4-benzothiadiazin-7-ol 1,1-dioxide

Dans un bicol purgé à l'azote et connecté à un piège contenant de l'eau de javel, on introduit au moyen d'une canule et en poussant à l'azote 100 ml (950 mmol) du complexe BF₃ Me₃S. Sous agitation et léger courant d'azote on additionne ensuite rapidement par petites portions une suspension de 5,63 g (21,8 mmol) du produit du stade B de la préparation I dans 75 ml de CH₂Cl₂. On stoppe le courant d'azote et agite la suspension réactionnelle une nuit à température ambiante. Le milieu réactionnel est refroidi dans un bain de glace et on y ajoute de la glace et de l'eau. La suspension est agitée 30 min, le précipité est filtré, rincé à l'eau et à l'heptane. Le solide est séché et recristallisé dans l'eau pour conduire au produit du titre.
*Point de fusion : 230-235 °C*
*Microanalyse élémentaire :*

| | *C* | *H* | *N* | *S* |
|---|---|---|---|---|
| *% théorique* | *44,26* | *3,71* | *11,47* | *13,13* |
| *% expérimental* | *44,55* | *4,18* | *11,34* | *13,59* |

### Stade B : 4-(2-Fluoroéthyl)-3,4-dihydro-2H-1,2,4-benzothiadiazin-7-ol 1,1-dioxide

Conditions et traitement identiques au stade C de la préparation 1 sauf que le produit attendu ne précipite pas après adition de HCl 1N mais est extrait par du CH₂Cl₂.
*Point de fusion : 178-180 °C*
*Microanalyse élémentaire :*

| | *C* | *H* | *N* | *S* |
|---|---|---|---|---|
| *% théorique* | *43,90* | *4,50* | *11,38* | *13, 02* |
| *% expérimental* | *43,73* | *4,37* | *11,10* | *12,80* |

### Préparation 3 : 4-(2-Chloroéthyl)-3,4-dihydro-2H-1,2,4-benzothiadiazin-7-ol 1,1-dioxide

### Stade A : 4-(2-Chloroéthyl)-7-méthoxy-4H-1,2,4-benzothiadiazine 1,1-dioxide

A une suspension de 1,0 g (3,90 mmol) du produit du stade A de la préparation I dans 20 ml de CH₂Cl₂ on ajoute, à température ambiante, 0,1 ml de DMF puis goutte à goutte une solution contenant 1,42 ml (19,5 mmol) de SOCl₂ dans 5 ml de CH₂Cl₂. A la fin de l'addition on obtient une solution qui est agitée au reflux du CH₂Cl₂ pendant 2 h. Le CH₂Cl₂ est évaporé sous vide et le résidu est repris dans une solution 5% de NaHCO₃. Après trituration du résidu, on obtient un solide qui est filtré, rincé par de l'eau, séché pour conduire au produit du titre.
*Point de fusion : 126-130 °C*
*Microanalyse élémentaire :*

| | *C* | *H* | *N* | *S* |
|---|---|---|---|---|
| *% théorique* | *43,72* | *4, 04* | *10, 20* | *11,67* |
| *% expérimental* | *43,79* | *4, 06* | *9,84* | *12, 01* |

### Stade B : 4-(2-Chloroéthyl)-7-méthoxy-3,4-dihydro-2H-1,2,4-benzothiadiazine 1,1-dioxide

Conditions et traitement identiques au stade C de la préparation 1.
*Point de fusion : 139-143 °C*
*Microanalyse élémentaire :*

| | *C* | *H* | *N* | S | *Cl* |
|---|---|---|---|---|---|
| *% théorique* | *43,40* | *4,73* | *10,12* | *12,59* | *12,81* |
| *% expérimental* | *43,73* | *5,05* | *9,89* | *11,07* | *13,30* |

### Stade C : 4-(2-Chloroéthyl)-3,4-dihydro-2H-1,2,4-benzothiadiazin-7-ol 1,1-dioxide

Conditions et traitement identiques au stade D de la préparation 1.
*Point de fusion : 171-173 °C*

### Exemple 1 : 3-Thiophènecarboxylate de 4-(2-fluoroéthyl)-1,1-dioxido-3,4-dihydro-2H-1,2,4-benzothiadiazin-7-yle

A une solution du produit obtenu dans la préparation 2 (200 mg, 0,812 mmol) dans 25 ml de CH₂Cl₂ on ajoute 228 µl (1,62 mmol) de Et₃N puis, goutte à goutte, une solution du chlorure de l'acide thiophène-3-carboxylique dans 10 ml de CH₂Cl₂. La solution réactionnelle est agitée 2h à température ambiante. On ajoute 20 ml de HCl 1N, décante la phase organique, sèche (MgSO₄), évapore sous vide. Le résidu est chromatographié sur silice (CH₂Cl₂/MeOH 98/2) pour conduire au produit du titre.
*Point de fusion : 183 °C*
*Microanalyse élémentaire :*

| | *C* | *H* | *N* | *S* |
|---|---|---|---|---|
| *% théorique* | *47,18* | *3, 68* | *7,86* | *17,99* |
| *% expérimental* | *46,91* | *3,85* | *7,62* | *18,20* |

### Exemple 2 : Benzoate de 4-(2-fluoroéthyl)-1,1-dioxido-3,4-dihydro-2H-1,2,4-benzothiadiazin-7-yle

On procède comme dans l'Exemple 1, à partir du composé obtenu dans la Préparation 2 et du chlorure de l'acide benzoïque.
*Point de fusion : 145 °C*
*Microanalyse élémentaire :*

| | *C* | *H* | *N* | *S* |
|---|---|---|---|---|
| *% théorique* | *54,85* | *4,32* | *8, 00* | *9,15* |
| *% expérimental* | *54,93* | *4,42* | *7,74* | *9,12* |

### ETUDE PHARMACOLOGIQUE DES PRODUITS DE L'INVENTION

### Etude des courants excitateurs induits par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.
L'enregistrement électrophysiologique est réalisé dans une chambre en plexiglass® à 20-24°C en milieu OR2 (J. Exp. Zool., 1973, 184, 321-334) par la méthode du «voltage-clamp» à 2 électrodes, une 3^{ème} électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b ― Résultats :

Les composés de l'invention potentialisent très fortement les effets excitateurs de l'AMPA et leur activité est très nettement supérieure à celle des composés de référence.

A titre d'exemple, le composé de l'Exemple 1 présente une EC2X de 8,0 µM.

**COMPOSITION PHARMACEUTIQUE**

| | |
|---|---|
| Formule de préparation pour 1000 comprimés dosés à 100 mg de 3-thiophènecarboxylate de 4-(2-fluoroéthyl)-1,1-dioxido-3,4-dihydro-2*H*-1,2,4-benzothiadiazin-7-yle (exemple 1) | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) : 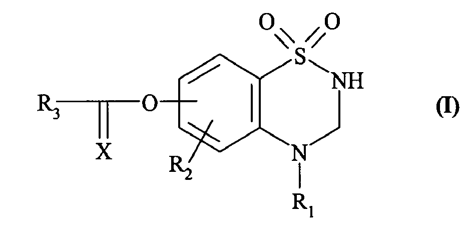 dans laquelle :
**X** représente un atome d'oxygène ou de soufre,
**R**_{**1**} représente un groupement alkyle (C₁-C₆) linéaire ou ramifié substitué par un ou plusieurs atomes d'halogène,
**R**_{**2**} représente un atome d'hydrogène ou d'halogène ou un groupement hydroxy,
**R**_{**3**} représente un groupement R₄ ou -Y-R₅ dans lesquels
R₄ représente un groupement alkyle (C₁-C₆) linéaire ou ramifié éventuellement substitué par un groupement aryle ; alkényle (C₂-C₆) linéaire ou ramifié éventuellement substitué par un groupement aryle ; polyhalogénoalkyle (C₁-C₆) linéaire ou ramifié ; cycloalkyle (C₃-C₇) ; adamantyle ; aryle ou hétéroaryle,
Y représente un atome d'oxygène ou de soufre ou un groupement NR dans lequel R représente un atome d'hydrogène ou un groupement alkyle (C₁-C₆) linéaire ou ramifié,
et R₅ peut prendre toutes les valeurs de R₄,
leurs énantiomères et diastéréoisomères ainsi que leurs sels d'addition à un acide ou à une base pharmaceutiquement acceptable.

2. Composés de formule (I), selon la revendication 1, pour lesquels X représente un atome d'oxygène.

3. Composés de formule (I), selon la revendication 1, pour lesquels R₁ représente un groupement halogénoéthyle.

4. Composés de formule (I), selon la revendication 1, pour lesquels R₂ représente un atome d'hydrogène.

5. Composés de formule (I), selon la revendication 1, pour lesquels R₃ représente un groupement aryle.

6. Composés de formule (I), selon la revendication 1, pour lesquels R₃ représente un groupement hétéroaryle.

7. Composés de formule (I), selon la revendication 1, qui sont :
• le 3-thiophènecarboxylate de 4-(2-fluoroéthyl)-1,1-dioxido-3,4-dihydro-2*H*-1,2,4-benzothiadiazin-7-yle, et
• le benzoate de 4-(2-fluoroéthyl)-1,1-dioxido-3,4-dihydro-2*H*-1,2,4-benzothiadiazin-7-yle.

8. Procédé de préparation des composés de formule (I), selon la revendication 1, **caractérisé en ce que** l'on utilise comme produit de départ le composé de formule (II) : 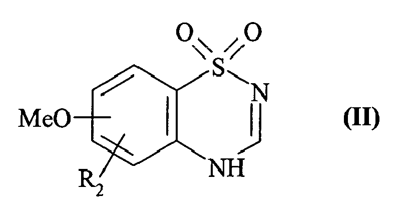 dans laquelle R₂ est tel que défini dans la formule (I),
sur lequel on condense, en milieu basique, un halogénoalkyle(C₁-C₆) linéaire ou ramifié comportant une fonction hydroxyle,
que l'on transforme ensuite en dérivé halogéné correspondant pour conduire au composé de formule (III) : 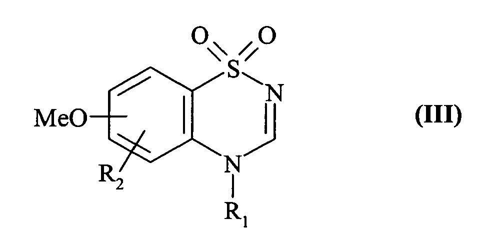 dans laquelle R₁ et R₂ sont tels que définis dans la formule (I),
que l'on soumet à une réduction, en présence de NaBH₄ par exemple, pour obtenir le composé de formule (IV) : 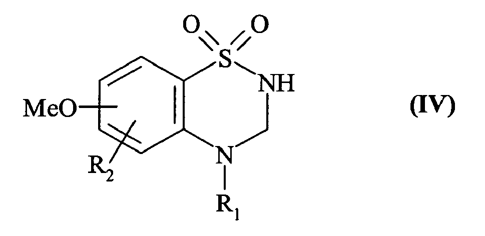 dans laquelle R₁ et R₂ sont tels que définis précédemment,
que l'on soumet à une réaction de déméthylation, en présence de BBr₃ ou BF₃ par exemple, pour conduire au composé de formule (V) : 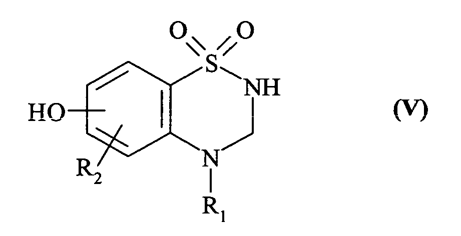 dans laquelle R₁ et R₂ sont tels que définis précédemment,
composé de formule (V) pouvant être également obtenu à partir du composé de formule (III) que l'on soumet à une réaction de déméthylation, en présence de BBr₃ ou BF₃ par exemple, pour conduire au composé de formule (VI) : 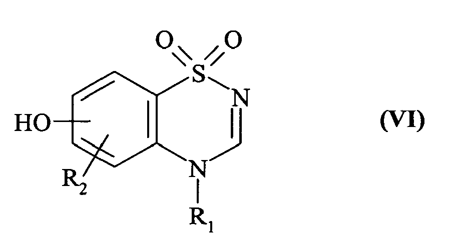 dans laquelle R₁ et R₂ sont tels que définis précédemment,
que l'on soumet à une réduction, en présence de NaBH₄ par exemple, pour obtenir le composé de formule (V) tel que défini précédemment,
sur lequel on condense le composé de formule (VII) : 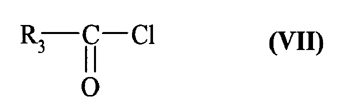 dans laquelle R₃ est tel que défini dans la formule (I)
pour obtenir le composé de formule (I/a), cas particulier des composés de formule (I) : 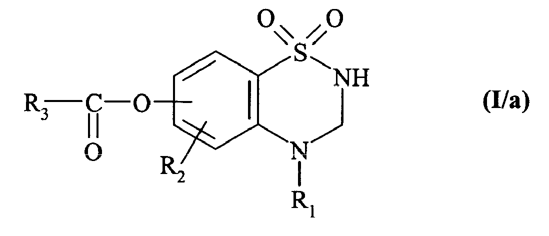 dans laquelle R₁, R₂ et R₃ sont tels que définis précédemment,
que l'on soumet à un agent de thionation, comme le réactif de Lawesson par exemple, pour conduire au composé de formule (I/b), cas particulier des composés de formule (I) : 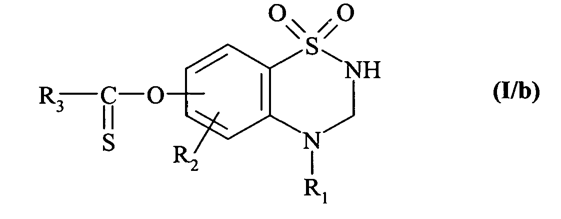 dans laquelle R₁, R₂ et R₃ sont tels que définis précédemment,
les composés de formule (I/a) et (I/b) formant l'ensemble des composés de formule (I) que l'on purifie, le cas échéant, selon une technique classique de purification, dont on sépare éventuellement les isomères, selon une technique classique de séparation, et que l'on transforme, si on le souhaite, en leurs sels d'addition, à un acide ou à une base pharmaceutiquement acceptable.

9. Composé et formule (V) selon la revendication 8 : 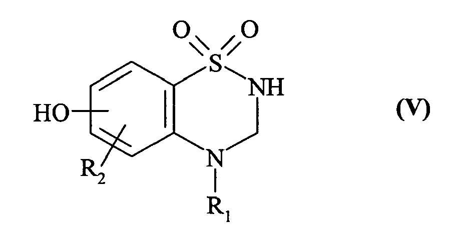 dans laquelle R₁ et R₂ sont tels que définis dans la revendication 8, utiles en tant qu'intermédiaires de synthèse des composés de formule (I).

10. Composé et formule (VIII), cas particulier des composés de formule (V) selon la revendication 9 : 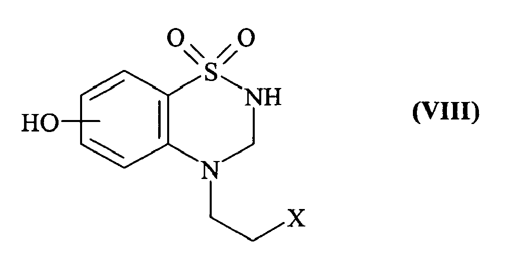 dans laquelle X représente un atome de fluor, de chlore, de brome ou d'iode, utile en tant qu'intermédiaire de synthèse des composés de formule (I).

11. Compositions pharmaceutiques contenant comme principe actif un composé selon l'une quelconque des revendications 1 à 7, 9 et 10 en combinaison avec un ou plusieurs excipients ou véhicules inertes, non toxiques, pharmaceutiquement acceptables.

12. Compositions pharmaceutiques selon la revendication 11 contenant comme principe actif un composé selon l'une quelconque des revendications 1 à 7, 9 et 10 utiles en tant que médicaments, comme modulateurs AMPA.
